# FASCICULE DE BREVET EUROPEEN

(11) **EP 2 209 797 B1**
(45) Date de publication et mention de la délivrance du brevet: **05.10.2016**
(21) Numéro de dépôt: 08871347.4
(22) Date de dépôt: 30.10.2008
(51) Int. Cl.: C07K 14/47, A61K 38/00, C07K 7/06, C07K 7/08

(54) **MODIFICATION DES SEQUENCES INTRINSEQUEMENT DESORDONNEES POUR LA PREPARATION DE VACCINS**
MODIFIKATION VON INTRINSISCH UNGEORDNETEN SEQUENZEN ZUR HERSTELLUNG VON IMPFSTOFFEN
MODIFICATION OF INTRINSICALLY DISORDERED SEQUENCES FOR PREPARING VACCINES

(30) Priorité: 31.10.2007 FR 0707646
(43) Date de publication de la demande: 28.07.2010
(73) Titulaire: Malina, Halina, 8952 Schlieren (CH)
(72) Inventeur: Malina, Halina, 8952 Schlieren (CH)
(86) Numéro de dépôt international: PCT/FR2008/001527
(87) Numéro de publication internationale: WO 2009/092891

(56) Documents cités:
- WO-A-00/58344
- WO-A2-03/045128
- WO-A2-2006/014638
- WO-A2-2007/005358
- FR-A- 2 901 479
- MALINA HALINA Z ET AL: "Xanthurenic acid translocates proapoptotic Bcl-2 family proteins into mitochondria and impairs mitochondrial function" BMC CELL BIOLOGY, vol. 5, no. April 6, 6 avril 2004 (2004-04-06), XP002521277 ISSN: 1471-2121 cité dans la demande
- PRILUSKY JAIME ET AL: "FoldIndex((c)): a simple tool to predict whether a given protein sequence is intrinsically unfolded" BIOINFORMATICS (OXFORD), vol. 21, no. 16, août 2005 (2005-08), pages 3435-3438, XP002521306 ISSN: 1367-4803

## Description

### LE CHAMP DE L'INVENTION

Le but de la présente invention est l'induction d'une réaction immunitaire supprimant la pathologie provoquée par des modifications de la physiologie cellulaire, dues à l'agrégation de séquences intrinsèquement désordonnées (IDSeq). L'invention concerne l'utilisation des séquences intrinsèquement désordonnées dans lesquelles un groupe secondaire, d'un ou plusieurs acides aminés, a réagi avec une petite molécule capable d'effectuer une polymérisation avec une autre IDSeq. Les polymères de l'IDSeq modifiée de façon covalente sont employés *in vivo* pour induire une réponse immunologique, afin de supprimer ou empêcher une maladie. Les anticorps obtenus par cette méthode sont employés pour la suppression de pathologies liées à un désordre métabolique présent lors d'infections ou de maladies métaboliques, le diagnostic ou les études sur le développement des maladies.

### BASES DE L'INVENTION

Le brevet de H. Malina EP001165600 a révélé une utilisation des protéines modifiées de façon covalente par l'acide xanthurénique pour la préparation de vaccin. L'acide xanthurénique, une petite molécule de la voie de la dégradation oxydative du tryptophane par l'indoleamine 2, 3-dioxygenase, modifie les protéines de façon covalente et imite le processus de modification covalente des protéines in vivo lors du vieillissement ou des infections. Le mécanisme de développement des maladies a été établi sur l'étude de l'acide xanthurénique dans une culture de cellules humaines et animales. L'interactome est un réseau régulé de protéines, où les interactions entre les protéines sont réversibles. L'invention est basée sur l'étude précédente montrant que les modifications covalentes des protéines en présence de l'acide xanthurénique mènent à l'apoptose pathologique (Malina et al. Physiologie de BMC 2001).
L'apoptose pathologique est provoquée par un réseau de protéines mal pliées, et est associée à la liaison de la calmoduline (Cam) à des régions de protéines régulées par la calmoduline. En parallèle, le phoshatidylinositol phosphate (PIP 2), ne peut plus réguler ces séquences occupées par la calmoduline. Le manque de signalisation de la calmoduline/PIP2 supprime la régulation de Bax par le PIP2, et mène à l'attachement covalente de Bax à la CAM, ainsi qu'à l'apoptose pathologique des cellules liée à l'activation constitutive du caspase-3 par l'intermédiaire de l'activation des caspase-9 mitochondrial (Malina H, non publié). *In vivo* et *in vitro,* la polymérisation des protéines est produite par de petites molécules comme l'acide xanthurénique, qui peuvent se lier de façon covalente aux protéines, et mener à leur polymérisation. Ces petites molécules présentes dans le corps humain peuvent provenir du métabolisme, de la pollution, mais aussi par exemple, de la thérapie endogène par de petites molécules. Ces molécules mènent à des interactions covalentes entre protéines. La molécule modèle pour étudier la formation du « misfoldome » *in vivo* et *in vitro* est l'acide xanthurénique, qui permet d'établir les interactions pathologiques entre protéines menant au développement des maladies, causant des dommages mitochondriaux (Malina et al BMC Cell Biology 2004.), un manque de régulation du calcium (Malina et al. BMC Ophthalmology 2002), l'interruption de *signaling* des protéines 14-3-3 (Malina et al. BBRC 2003). Les protéines modifiées ont de nouvelles interactions covalentes avec d'autres protéines, et changent leur place et leur rôle dans la cellule. Les séquences ayant un groupe de trois acides aminés basiques sont modifiés préférentiellement par les petites molécules de façon covalente, et peuvent être employées pour la préparation d'anticorps et la modification du réseau de calmoduline et de phosphate du phosphatidylinositol (Malina H., Patent Fr 0604671). La présente invention a révélé la méthode de préparation d'anticorps contre les peptides polymérisés, qui correspondent à la région appelée IDSeq, laquelle modification est responsable du développement des maladies.
De nombreuses régions des protéines se sont avérées être intrinsèquement désordonnées. Les régions intrinsèquement désordonnées sont cruciales à la fonction des protéines, particulièrement celles impliquées dans la signalisation et la régulation (Chen W.J. et al. J. Proteome Res.5, 879-887, 2006). L'identification des régions des protéines est très importante pour la prévision de leur structure et de leur caractérisation fonctionnelle. Les régions des protéines, mal structurées intrinsèquement, jouent un rôle clef dans la signalisation des cellules, la dégénérescence des cellules, et le cancer (Jakoucheva LM, CJ Brown , Lawson JD, Obradowic Z, Dunker AK, J. Mol. Biol. 323, 573-584, 2002). L'« unfoldability » des séquences est provoqué par la charge élevée de ces séquences riches en lysine, arginine, histidine et glutamine présentes dans l'IDSeq. Les IDSeq des protéines sont détectées par exemple par le logiciel FoldIndex© (Jaime Prilusky, Tzviya Zeev-Ben-Mordehai, Edwin Rydberg, Clifford Felder, Israel Silman et Joel L. Sussman, basé sur l'algorithme proposé par (Jaime Prilusky, Tzviya Zeev-Ben-Mordehai. Edwin Rydberg, Clifford Felder, Israel Silman et Joel L. Sussman, basé sur l'algorithme proposé par Uversky VN, Gillespie JR, and Fink AL. Why are "natively unfolded" proteins unstructured under physiologie conditions? Proteins 2000; 41:415-427)

L'invention est basée sur la découverte montrant que l'apoptose liée au vieillissement et aux infections, appelées apoptoses pathologiques, est provoquée par des protéines modifiées de façon covalente. Les protéines modifiées de façon covalente changent de localisation dans la cellule en raison des nouvelles interactions covalentes entre protéines. Au contraire, l'état physiologique est assuré par un réseau de protéines électrostatiquement liées, l'interactome. Les interactions entre protéines dans l'interactome sont flexibles afin d'assurer la régulation des protéines. La flexibilité est assurée par les régions de la protéine responsables de sa régulation, et se nomment les séquences intrinsèquement désordonnées. La pathologie des cellules est causée par ces séquences (les IDSeq), qui sont modifiées de façon covalente, et ne peuvent plus participer à la régulation. Cette invention révèle une nouvelle technologie de préparation des vaccins en utilisant ces séquences modifiées et polymérisées, qui peuvent être employées pour détecter le développement précoce des maladies, pour les empêcher ou les guérir. Les segments des séquences caractérisés comme segment intrinsèquement désordonnées, définies par exemple grâce à FoldIndex©, correspondant aux séquences utilisées pour le développement de médicaments dans la présente invention.
Cette méthode révèle la préparation des vaccins, en utilisant un polymère de l'IDSeq modifiée de façon covalente, à savoir la pIDSeqC. Les pIDSeqC ne peuvent pas jouer un rôle de normalisation, et induisent d'ailleurs une nouvelle et forte signalisation, recouvrant la signalisation de l'interactome. Les nouvelles interactions covalentes entre les protéines mènent à la pathologie des cellules et aux désordres métaboliques associés au vieillissement ou aux infections.
La thérapie ou la prévention des maladies selon cette invention est faite par l'élimination des pIDSeqC, car « l'unfoldability » est nécessaire à l'état physiologique normal des mammifères. La vaccination passive ou active avec le pIDSeqC supprime une pathologie provoquée par ces interactions covalentes. L'invention diffère de la thérapie courante, qui vise les protéines ou les peptides normaux. L'invention fournit davantage de possibilités de thérapie par induction de la réponse immunologique contre les interactions covalente entre IDSeqC.

### DESCRIPTION DE L'INVENTION

Différents termes reliés aux méthodes et à d'autres aspects de la présente invention sont utilisés dans les spécifications et les revendications.
Le terme **"petites molécules polymérisant"** tient pour toute molécule capable de réagir avec un groupe secondaire d'acides aminés, et conduisant à la polymérisation d'un, deux ou plusieurs peptides.
**Le terme « protéines mal pliées** » signifie que les protéines ont été modifiées de manière covalente, de sorte qu'une petite molécule, un lipide ou une protéine régulatrice ne puissent avoir d'interaction électrostatique avec cette séquence.
**La modification covalente de l'IDSeq** signifie que les acides aminés chargés sont modifiés de manière à ce qu'ils deviennent non accessibles, et ce de manière définitive, pour une petite molécule ou une protéine de régulation électrostatique.
**La polymérisation** de l'IDSeqC signifie que les IDSeqC, appartenant à la même ou à différentes protéines, forment un dimére ou un polymère. La modification covalente des peptides peut avoir comme conséquence d'agrégation des peptides, où un ou plusieurs peptides et une ou plusieurs petites molécules sont utilisées pour la préparation de l'agrégat.
Les termes "protéines polymérisées", "protéines agrégées" et "protéines cross-linquées" sont employées indifféremment ci-dessous.
**La vaccination on l'immunisation** contre les protéines mal pliées consiste en l'introduction des séquences pIDSeqC dans l'organisme vivant, par tout moyen connu comme par exemple: une application sublinguale, nasale, dermique ou une injection dans un organisme vivant un anticorps obtenu par l'utilisation de pIRSeqC comme antigène.
**La thérapie** représente tout succès d'amélioration du bien-être physique ou mental d'un être humain, par l'atténuation de n'importe quel processus dégénératif induit par une infection, un trauma ou le vieillissement, comprenant, sans limitation mais par exemple le vieillissement de la peau, l'anxiété, la dépression, la circulation du sang, la diminution de la vision, la dégénération neurologique, les maladies du coeur, les affections hépatiques, l'ostéoporose, l'immunosuppression, les cancers.
**"Le milieu pharmaceutiquement acceptable"** se rapporte à un milieu qui n'interfère pas avec l'efficacité de l'activité biologique du ou des ingrédients actifs, et n'est pas toxique pour l'organisme auquel il est administré.
**« Predicted disorder segment** » est fragment de séquence, qui doit rester accessible pour une régulation et dans les cas des modifications covalentes conduites à des maladies. Anticorps contre ces séquences modifies sont utilisé pour thérapie.
**L'IRSeq** mentionné dans la présente invention signifie qu'un peptide est estimé, par le logiciel de FoldIndex©, comme segment intrinsèquement désordonné. L'immunisation se rapporte à une induction d'immunité chez l'animal ou l'humain, par tout moyen accessible, en utilisant le polymère IRSeqC.
La vaccination vise la pathologie provoquée par les séquences polymérisées. Beaucoup de maladies sont associées à la dégénérescence des tissus, qui est provoquée par l'apoptose pathologique. L'apoptose pathologique est provoquée par les interactions covalentes entre les protéines. A l'inverse, l'apoptose observée dans l'homéostasie de tissus et le développement, est causée par des interactions physiologiques. Selon cette invention, la thérapie des maladies est possible par une réaction immunitaire contre les pIDSeqC des protéines. Ces protéines modifiées peuvent être protéines membranaires (surtout G-protéines et GPRC), des protéines cytoplasmiques ou des protéines nucléaires (par exemple RAR récepteurs etc.). La modification covalentes des protéines comme précurseur de prion, les protéines précurseur du bêta amyloïde, le CD 19, l'EGFR, le VEGFR, les protéines suppresseurs des tumeurs comme par exemple la protéine de régulation du suppresseur du gliomes tumeur, la protéine p53, les protéines RGS, les récepteurs de chemokines, et interleukines conduites à leur agrégation (polymérisation) qui exerce un nouveau *signaling* qui est pathologique.
Les protéines sont polymérisées par de petites molécules (exemple endogène de l'acide xanthurénique ou des médicaments comme l'Endoxan, des petites molécules de pollution, de la fumée de cigarette), qui modifient les de façon covalentes irréversible les fonctions amines secondaires des acides aminés.
Un important mécanisme menant aux maladies, est la diminution de la production du PIP2, due à la polymérisation de l'IDSeqC dans les membranes, menant à une perte d'activité du PI-4 kinase et du PI-5 kinase, et à une polymérisation covalente des IDSeqC de la PI3 kinase p85 et p100. L'interaction de l'IDSeqC de ces kinases avec les liens phosphatidylinositol phosphates aux protéines, comme le gelsolin, le PLC, l'EGFR, l'adducin etc. rend impossible une régulation des ces protéines par les PIP2. De la même manière, la polymérisation de l'IDSeqC des protéines régulées par les PIP2, avec une séquence des G-protéines ou n'importe quelle protéine associée au réseau de G-protéine, suppriment la signalisation physiologique, et causent les maladies. La plateforme de technologie pour la préparation des médicaments, selon cette invention, consiste à choisir, par le software FoldIndex©, un ou plusieurs IDSeq d'une protéine suspectée d'être impliquée dans une maladie. L'antigène préparé par la polymérisation des peptides est injecté dans un organisme vivant pour induction une réponse immunitaire. L'efficacité thérapeutique de l'anticorps présent dans le sérum de l'animal, peut être estimée sans davantage de purification, en l'utilisant dans une culture de cellules en présence d'acide xanthurénique. L'anticorps peut ensuite être purifié par toute méthode de purification d'anticorps, et amélioré par toute méthode technologique. Par exemple, l'anticorps polyclonal purifié par une chromatographie d'affinité est un vaccin thérapeutique passive efficace, car correspondant à la situation *in vivo* de la formation des protéines menant aux maladies.

### Description détaillée

Les interactions électrostatiques entre des protéines dans les membranes, et en particulier dans les « lipid rafts », sont responsables de la régulation de la signalisation dans les cellules. Un des événements en amont de la pathologie cellulaire, sont les interactions covalentes des protéines impliquées dans le réseau des protéines, des interactions de GPCR, de la synthèse des phosphates de phospholipides, de 14-3-3 protéines, des protéines régulées par la calmoduline, ainsi que des protéines des membranes, comme la protéine précurseur de bêta amyloïde, des protéines de précurseur de prion, du PAR, de l'adducin, des protéines régulatrices des gènes suppresseurs du cancer, des récepteurs pour des chemokines, des interleukines, du neuregulin, de l'EGFR, du VGFR, du TGFR, etc. L'invention décrit une technologie de préparation des médicaments en utilisant des peptides modifiés de façon covalente. Le peptide est synthétisé *in vitro*, et ses groupes d'aminés secondaires sont laissés libres pour modification. Le ou les peptides peuvent être modifiés par n'importe quelle méthode, mais l'incubation, avec une solution de substance polymérisante, est une méthode satisfaisante. La période et la condition de la modification dépendent du degré de modification à réaliser. La substance polymérisant est ajoutée au peptide, de préférence dans un rapport molaire correspondant au nombre d'acides aminés à modifier dans le peptide. Les pIDSeqC sont employés comme antigène, pour induire une réaction immunitaire. La présence d'un groupe phosphorylé, contenant une modification de peptide, change la caractéristique de l'anticorps produit. La génération d'anticorps contre une polymérisation de protéines est identique à la situation lors du développement des maladies associées au vieillissement ou aux infections. Les anticorps contre l'pIDSeqC ont des activités thérapeutiques *in vitro* et *in vivo,* et stoppent la pathologie chez l'humain et l'animal. Ces peptides mal pliés sont employés pour la vaccination de mammifères, de préférence un animal. L'humain est de préférence traité par un anticorps obtenu contre l'IDSeqC dans un animal, pour éviter une production non contrôlée de l'anticorps dans l'humain. Cependant, certains antigènes, et dans certaines situations cliniques particulières, peuvent être employés directement dans l'humain comme vaccin.

L'anticorps peut agir directement sur les cellules, pour bloquer des protéines mal pliées dans la membrane des cellules et prévenir la pathologie, et, par exemple, mener à une protection contre l'apoptose pathologique dans une culture de cellules ou une blessure. L'anticorps peut être polyclonal, « single chain », anticorps de recombinaison ou préparé par n'importe quel moyen présent ou futur menant à la production d'un anticorps ou de sa partie active.
Les anticorps préparés sur cette plateforme bloquent *in vivo* les réseaux de protéines modifiées de façon covalente, dans la même manière que l'utilisation de l'anticorps supprimera la signalisation des interactions incorrectes menant aux maladies. L'acide xanthurénique est une substance modèle, très commode pour la polymérisation des peptides, car soluble dans l'eau, colorée en jaune, et fluorescente. La liaison covalente de l'acide xanthurénique - ou de tout dérivé d'acide xanthurénique - avec les peptides, imitent la situation de la modification in vivo. Cette approche mène à la préparation d'anticorps efficaces contre les peptides mal pliée, qui suppriment la pathologie *in vivo.* La chromatographie sur couche mince et la spectrométrie de masse peuvent être utilisés pour surveiller la modification du peptide. L'anticorps contre le pIDSeqC est produit dans un animal, de préférence par trois injections faites à l'animal par intervalle d'un mois ou de 6 semaines. Le sérum de l'animal a une activité thérapeutique, et mène à une suppression de la pathologie des cellules dans une culture de cellules primaires, chez les humains et chez les animaux. Pour une activité biologique plus élevée, le sérum est purifié par chromatographie d'affinité sur tout support chromatographique accessible. Les anticorps contre le pIDSeqC ont une activité thérapeutique *in vitro* et *in vivo,* et stoppent la pathologie chez les humains et les animaux. L'anticorps peut être livré de n'importe quelle manière connue, de préférence par application ou injection pour une thérapie de l'humain, par injection pour une thérapie de l'animal. Les anticorps peuvent agir sur le blocage des protéines cellulaire mal plié dans la membrane de cellules, conduisant à une protection contre les pathologies. Les exemples ci-dessous montrent la préparation des vaccins en utilisant l'IRSeqC.
Il est revendiqué une utilisation des séquences modifiées des protéines pour la fabrication des cosmétiques, du diagnostique et de la thérapie des maladies.

### Exemple 1.

Une incubation d'un peptide, ayant l'IDSeq, avec l'acide xanthurénique, a conduit à la modification covalente de ce peptide et à sa dimérisation (pIDSeqC) observée par le MS. Le produit de réaction, nommé pIDSeqC, a été utilisé comme antigène. 1 ml contenant 400 microgramme de peptide modifié dans 1 ml 7.2 tampon phosphates a été mélangé avec 1 ml d'adjuvant de Freund. Les lapins ont été injectés trois fois, une fois toutes les six semaines. Les lapins ont produit l'anticorps souhaité, ainsi contre la séquence modifiée par l'acide xanthurénique, comme il a été démontré par l'analyse de Western blot, car l'anticorps a reconnu la séquence dans un échantillon de protéines qui ont été cultivées, en présence de l'acide xanthurénique, mais non dans des cultures de contrôle. Un protocole standard a été utilisé pour la préparation du plasma, et l'anticorps a été purifié du plasma par Sepharose conjuguée avec des protéines G. L'immunoprécipitation de protéines par un anticorps anti-pIDSeqC, et leur analyse par Western blots ont été utilisées pour des études de pathologies cellulaires. Les anticorps introduits dans une culture cellulaire ont bloqué les interactions pathologiques causées par l'acide xanthurénique.
Les anticorps ont montré une accumulation des séquences modifiées dans le sang permettant le diagnostique précoce des maladies causées par la modification de ces séquences.
Une immunisation passive *in vivo* (humains et animaux), a conduit à l'élimination des ces séquences modifiées, et une suppression de pathologies induites par un mauvais fonctionnement de ces protéines.

### Exemple 2.

130 kDa phosphatidylinositol 4,5-biphosphate-dependent ARF1 GTPase-activating protéine (Q9ULH1) La séquence de cette protéine, nommée plus bas la séquence 1, a été synthétisée et modifiée comme décrit dans l'Exemple 1.

La séquence 1:
320-N KEYGSEKKGY LLKKSDGIRK VWQRRKCSVK N- 351 (SEQ ID NO : 1)
Predicted disorder segment: [1]-[32] length: 32 score: -0.47 ± 0.00

Anticorps contre la séquence ont établi la synthèse des PIP2 dans la cellule.

### Exemple 3.

### B-lymphocyte antigen CD19 (P15391)

Séquence 2 :
316 LVLR RKRKRMTDPTRRFFKV 335 (SEQ ID NO : 2)
Predicted disorder segment: [1]-[20] length: 20 score: -0.71 ± 0.00

Anticorps contre la séquence 2-pIDSeqC, préparé comme décrit dans le Exemple 1 prévient la accumulation de lymphocytes ayant cette séquence dans le sang.

### Exemple 4

### 130 kDa phosphatidylinositol 4,5-biphosphate-dependent ARF1 GTPase-activating protéine (Q9I3LH1).

La séquence 3 :
NKEYGSEKKG YLLKKSDGIR KVWQRRKCSV KNGIL (SEQ ID NO : 3)
   Predicted disorder segment: [1]-[35] length: 35 score: -0.42 ± 0.08

Anticorps contre la séquence 3-pIDSeqC, ont été préparé comme décrit dans le Exemple 1.
Anticorps ont prévenu l'apoptose pathologique.

### Exemple 5

### ROCK2_HUMAN Rho-associated protein kinase 2 (075116)

Séquences 4 et 5.
1165 TKKFGWVKKY V 1175 (SEQ ID NO : 4)
   Predicted disorder segment: [1]-[11] length: 11 score: -0.27 ± 0.12
1344 VKKIPKKP 1352 (SEQ ID NO : 5)
   Predicted disorder segment: [1]-[8] length: 8 score: -0.79 ± 0.29

Anticorps contre les séquences 4 et 5-pIDSeqC, ont été préparé comme décrit dans le Exemple 1.
Anticorps ont prévenu modification de ROCK2.

### Exemple 6.

### Phosphatidylinositol-3,4,5-trisphosphate 3-phosphatase and dual-specificity protein phosphatase PTEN(Q6XPS3)

Les séquences 6 et 7.
257 FHKQNKMLKK DKMFHF 271 (SEQ ID NO : 6)
   Predicted disorder segment: [1]-[16] length: 16 score: -0.57 ± 0.27
158 RDIYETDYYRKGGK 171 (SEQ ID NO : 7)
   Predicted disorder segment: [1]-[14] length: 14 score: -0.66 ± 0.18

Anticorps contre les séquences 5-pIDSeqC, ont été préparé comme décrit dans le Exemple 1.
Anticorps ont prévenu PTEN anormalité.

### Exemple 7.

### EGFR récepteur (P00533)

Séquence 8: (SEQ ID NO : 8)
216 NCQKLTKIIC AQQCSGRCRG KSPSDCCHNQ CAAGCTGPRE SDCLVCRKFR 251 DEATCKDTCP PLMLYNPTTY QMDVNPEGKY SFGATCVKKC PRN 299

Predicted disorder segment: [1-[5] length: 5 score: -0.29 ± 0.09
Predicted disorder segment: [14]-[27] length: 14 score: -0.40 ± 0.26
Predicted disorder segment: [36]-[41] length: 6 score: -0.36 ± 0.20
Predicted disorder segment: [47]-[54] length: 8 score: -0.43 ± 0.19
Predicted disorder segment: [67]-[81] length: 15 score: -0.25 ± 0.15
Predicted disorder segment: [86]-[93] length: 8 score: -0.38 ± 0.43

Anticorps contre les séquences 8-pIDSeqC, ont été préparé comme décrit dans le Exemple 1.
Anticorps ont prévenu la signalisation anormale du récepteur modifié.

### Exemple 8

### Pro-neuregulin-1 (Q02297)

### (Glial growth factor) observed in breast cancer.

Séquence 9 :
0 MSERKEGRGK GKGKKKERGS GKKPESA 27 (SEQ ID NO: 9)

Anticorps contre la séquence 9-pIDSeqC, ont été préparé comme décrit dans le Exemple 1.
Anticorps ont prévenu le nucleus anormalités.

### Exemple 9

### 14-3-3 protéine gamma (P61981)

Séquence 10 :
75 EKKIE MVRAYREKIE KELEAV 96 (SEQ ID NO : 10) Predicted disorder segment: [1]-[8] length: 8 score: -0.28 ± 0.15

Anticorps contre la séquence 10-pIDSeqC, ont été préparé comme décrit dans le Exemple 1.
Anticorps ont prévenu l'apoptose pathologique.

### Exemple 10

### Actin-depolymerizing factor (P06396)

Séquence 11 :
161 FKSGLKYKKG 170 (SEQ ID NO: 11) Predicted disorder segment: [1]-[10] length: 10 score: -0.53 ± 0.00

Anticorps contre la séquence 11-pIDSeqC, ont été préparé comme décrit dans le Exemple 1. Anticorps ont prévenu l'apoptose pathologique.

### Exemple 11

### Phosphatidylinositol-3, 4, 5-trisphosphate 3-phosphatase TPTE2 Synonyms EC 3.1.3.67, TPTE and PTEN homologous inositol lipid phosphatase

Séquences 12
250 VRFLDKKHRN HYRVYNLCSE RA 271 (SEQ ID N° 12) Predicted disorder segment: [1]-[14] length: 14 score: -0.64 ± 0.28

Anticorps contre la séquence 12 pIDSeqC, ont été préparé comme décrit dans le exemple 1. Anticorps ont prévenu la modification de TPTE2

### Exemple 12

### Rho/Rac guanine nucleotide exchange factor 2 (P05067)

Séquences 13:
WCKRGRKQCK THPHF 195 (SEQ ID N°13) Predicted disorder segment: [1]-[15] length: 15 score: -0.64 ± 0.00
FQKAKERLEA KHRERMSQVM REW 440 (SEQ ID N°14) Predicted disorder segment: [1]-[23] length: 23 score: -0.46 ± 0.14

Anticorps contre les séquences 13 et 14 pDSeqC, ont été préparé comme décrit dans le Exemple 1. Anticorps ont prévenu l'apoptose pathologique.

### Exemple 13

Amyloid beta A4 precursor protein-binding family B member 1-interacting protein (Q7Z5R6)
La séquence 15 : 159 AKA DKIKLALEKL KEAKVKKLV 180 (SEQ ID NO :15)

Anticorps contre la séquences 15 pDSeqC, ont été préparé comme décrit dans le Exemple 1. Anticorps ont prévenu l'apoptose pathologique et beta-amyloide agrégation.

### Exemple 14

### Brain-specific angiogenesis inhibitor 1-associated protein 2 BAIP2_HUMAN (Q9UQB8) Insulin receptor substrate p53

La séquence 16 :
Predicted disorder segment: [1]-[43] length: 43 score: -0.48 ± 0.23 ALKKYQTEQR SKGDALDKCQ AELKKLRKKS QGSKNPQKYS DKE (SEQ ID NO : 16)

Anticorps contre la séquence 16 pIDSeqC, ont été préparé comme décrit dans le Exemple 1. Anticorps ont prévenu l'apoptose pathologique et insulin la modification de récepteur de la insuline.

### Exemple 15

### HUMAN Q59GZ4

La séquence 17:
Molecular function: protein serine/threonine kinase activity NRKDFKIDRK KA (SEQ IN NO : 17)
Predicted disorder segment: [1]-[12] length: 12 score: -0.77 ± 0.15

Anticorps contre la séquence 17 pIDSeqC, ont été préparé comme décrit dans le Exemple 1. Anticorps ont prévenu l'apoptose pathologique et kinase modification.

### Exemple 16.

### Rho family, small GTP binding protein Racl (P 63000)

Séquence 18 :
181 P VKKRKRKCL 190 (SEQ ID N : 18)

Anticorps contre la séquence 18 pIDSeqC, ont été préparé comme décrit dans le Exemple 1. Anticorps ont prévenu l'apoptose pathologique.

### Exemple 17.

Breast cancer nuclear receptor-binding auxiliary protein (Q 12802).

Séquences 19, 20, 21:
TRLFGLTKPK EKKEKKKKNK TSRSQPGDGP A 2801 (SEQ ID NO:19)
FS YIKNKMSSSK KSKEKEKEKD KIKEKEKDSK DKEKDKKTVN GHTF 1794 (SEQ ID NO : 20)
NTDRSCR KKNKGVERKG E 381(SEQ ID NO : 21)

Anticorps contre les séquences 19, 20, 21 pIDSeqC, ont été préparé comme décrit dans le Exemple 1. Anticorps ont prévenu anormalité du nucleus.

### Exemple 18

### A-Raf proto-oncogene serine/threonine-protein kinase Synonyms EC 2.7.11.1

Séquences 22:
261 ASVSSGRKSP HSKSPAEQRE RKSLADDKKK VKNLGYRD 297 (SEQ ID NO : 22)
   Predicted disorder segment: [1]-[38] length: 38 score: -0.52 ± 0.17

Anticorps contre la séquence 22 pIDSeqC, ont été préparé comme décrit dans le Exemple 1. Anticorps ont prévenu anormalité de la kinase.

### Exemple 19

### ABl kinase

Séquences 23 et 24:
1 EESRVRRHKH SSESPGRDKG (SEQ ID NO : 23)
   Predicted disorder segment: [1]-[20]length: 20 score: -0.67 ± 0.26
FLRRKRD (SEQ ID NO : 24)
   Predicted disorder segment: [1]-[7] length: 7 score: -0.67 ± 0.41

Anticorps contre les séquences 23 et 24 pIDSeqC, ont été préparé comme décrit dans le Exemple 1. Anticorps ont prévenu anormalité du nucleus.

### Exemple 20

### Tubby protein (P 50607)

La séquence 25:
122 ARKEKKGK HKG 132 (SEQ ID NO : 25)

Anticorps contre les séquences 25 pIDSeqC, ont été préparé comme décrit dans le Exemple 1. Anticorps ont prévenu anormalité du cytosquelette.

### Exemple 21

### Myosin phosphatase Rho-interacting protein (Q6WCQ1).

La séquence 26 et 27 :
152 NKQNQKKKRK V 162 (SEQ ID NO : 26) Predicted disorder segment: [1]-[11] length: 11 score: -1.48 ± 0.21
581 ERERARRREE RRKRF 585 (SEQ ID NO : 27) Predicted disorder segment: [1]-[15] length: 15 score: -1.23 ± 0.19

Anticorps contre les séquences 26 et 27 pIDSeqC, ont été préparé comme décrit dans le Exemple 1. Anticorps ont prévenu l'apoptose pathologique et établi la phosphorylation de la myosine.

### Exemple 22

### PtdIns(5)P-4-kinase isoform 2-alpha (P 48426).

Les séquences 28, 29, 30:
18 ASKTKTKKKH FVAQKVKLF 31 (SEQ ID NO : 28)
153 EMHNILK KYHQYIVECH GI 172 (SEQ ID NO : 29)
365 AKKKA AHAAKTVKHG A 381 (SEQ ID NO : 30)

Anticorps contre la séquence 28, 29 et 30 pIDSeqC, ont été préparé comme décrit dans le Exemple 1. Anticorps ont prévenu l'apoptose pathologique.

### Exemple 23

### VGFR 1 (P17948)

Les séquences 31:
345 VKHRK QQVLETVAGK RSYRLSMKVK 360 (SEQ ID NO : 31)

Anticorps contre la séquence 31 pIDSeqC, ont été préparé comme décrit dans le Exemple 1. Anticorps ont prévenu formation des nouveaux vaisseaux.

### Exemple 24

### Alpha-Adducin (P 35611)

Les séquences 32, 33,34, 35, 36, 37, 38 :
EKYK AKSRSPGSPV 360 (SEQ ID NO : 32)
EKYKAKSRSPGSPV 14 residues, unfoldability -0.437 (Charge: 0.214, Phobic: 0.333)
REKSKKYS 8 residues, unfoldability -1.008 (Charge: 0.375, Phobie: 0.186) (SEQ ID NO : 33)
L REKSKKYSDV 410 (SEQ ID NO : 34)
E RKQKGSEENL 590 (SEQ ID NO : 35)
ERKQKG 6 residues, unfoldability -1.108 (Charge: 0.333, Phobic: 0.135) (SEQ ID NO : 36)
PGKSPSKKKK KFRTPSFLKK SKKKSDS 730 (SEQ ID NO : 37)
GKSPSKKKKKFRTPSFLKKSKKK 23 residues, unfoldability -0.892 (Charge: 0.522, Phobic: 0.280) (SEQ ID NO:38)

Anticorps contre les séquences 32, 33, 34, 35, 36, 37, 38 pIDSeqC, ont été préparé comme décrit dans le Exemple 1. Anticorps ont prévenu dégénérescence de cytosquelette dans une culture cellulaire.

### Exemple 25

### Centrosome-associated actin homolog (P61163)

Les séquences 39 et 40:
VSKKEYEEDG ARSIHRKTF 376 (SEQ ID NO : 39) Predicted disorder segment: [1]-[19] length: 19 score: -0.28 ± 0.00
MYRRKSKQAL RDYKKVQIQL EN (SEQ ID NO : 40) Predicted disorder segment: [1]-[22] length: 22 score: -0.46 ± 0.00

Anticorps contre les séquences 39 et 40 pIDSeqC, ont été préparé comme décrit dans le Exemple 1. Anticorps ont prévenu formation les cellules avec multiples nucleus.

### Exemple 26.

### Regulator of G-protein signalling 12 (A2A496)

Les séquences 41 et 42:
219 PKKLSGKSKSGRSLNEELGDEDSEKKRKGAFFSWSRTRSTGRSQKKREHGDH A 271 (SEQ ID NO: 41) Predicted disorder segment: [1]-[53] length: 53 score: -0.54 ± 0.23
499 NS IKIKGENGKN ARDPRLSKRE ESIAKIGKKK YQKIN 535 (SEQ ID NO : 42) Predicted disorder segment: [1]-[37] length: 37 score: -0.44 ± 0.15

Anticorps contre les séquences 41et 42 pIDSeqC, ont été préparé comme décrit dans le Exemple

### 1. Anticorps ont prévenu l'apoptose pathologique.

### Exemple 27.

### Heat shock protein HSP 90-alpha (P07900).

Les séquences 43:
267 EKK DGDKKKKKKI KEKYIDQEEL-290 (SEQ ID NO : 43)

Anticorps contre la séquence 43 pIDSeqC, ont été préparé comme décrit dans le Exemple 1. Anticorps ont aboli la anormalité du nucleus.

### Exemple 28.

### T-cell activation Rho GTPase-activating protein (Q8N103)

Les séquences 44 et 45 a, b :
VQGKTKRPVD LKIKNL (SEQ ID NO : 44)
   Predicted disorder segment: [1]-[14] length: 14 score: -0.45 ± 0.15
VSRLVKKIPK KPPA (SEQ ID NO : 45)
   Predicted disorder segment: [5]-[14] length: 10 score: -0.53 ± 0.22

Anticorps contre les séquences 44 et 45 pIDSeqC, ont été préparé comme décrit dans le Exemple 1. Anticorps ont prévenu l'apoptose pathologique.

### Exemple 29.

### Rho/Rac guanine nucleotide exchange factor 2 (Q92974).

Les séquences 46 et 47:
WCKRGRKQCK THPHF 200 (SEQ ID NO : 46)
   Predicted disorder segment: [1]-[23] length: 23 score: -0.46 ± 0.14
FQKAKERLEA KHRERM 433 (SEQ ID NO : 47)
   Predicted disorder segment: [1]-[16] length: 16 score: -0.56 ± 0.30

Anticorps contre les séquences 46 et 47 pIDSeqC, ont été préparé comme décrit dans le Exemple 1. Anticorps ont prévenu l'apoptose pathologique.

### Exemple 30.

### Rho kinase 2 (O 507516).

La séquence 48.
62 LRKNKNIDNF LNRYEKIVKK IRG 85 (SEQ ID N°29) Predicted disorder segment: [1]-[7] length: 7 score: -0.66 ± 0.39

Anticorps contre la séquence 48 pIDSeqC, ont été préparé comme décrit dans le Exemple 1. Anticorps ont prévenu l'apoptose pathologique.

### Exemple 31

### RAR

### Retinal-specific ATP-binding cassette transporter.

La séquence 49 :
350 EKKKKITV 363 (SEQ ID NO : 49) Predicted disorder segment: [1]-[5] length: 5 score: -0.54 ± 0.00

Anticorps contre la séquence 49 pIDSeqC, ont été préparé comme décrit dans le Exemple 1. Anticorps ont aboli la anormalité du nucleus.

### Exemple 32

### ARF-GAP with GTP-binding protein-like, ankyrin repeat and pleckstrin homology domains 1 (Q9UPQ3).

La séquence 50 :
524 NRKKHR RKKSTSNFKA 540 (SEQ ID NO : 50) Predicted disorder segment: [1]-[16] length: 16 score: -1.12 ± 0.21

Anticorps contre la séquence 50 pIDSeqC, ont été préparé comme décrit dans le Exemple 1. Anticorps ont prévenu l'apoptose pathologique.

### Exemple 33

### Receptor interleukin I beta (P01584)

Les séquence 51, 52, 53 a, b, c :
F RGRHYKREFR 40 (SEQ ID NO : 51)
   Predicted disorder segment: [1]-[11] length: 11 score: -0.95 ± 0.26
LRIKK KKE 230 (SEQ ID NO : 52)
   Predicted disorder segment: [1]-[8] length: 8 score:-1.08 ± 0.29
H RRCKHRTGKA 380 (SEQ ID NO : 53)
   Predicted disorder segment: [1]-[11] length: 11 score: -0.98 ± 0.29

Anticorps contre les séquences 51, 52, 53 pIDSeqC, ont été préparé comme décrit dans le

### Exemple 1. Anticorps ont prévenu l'apoptose pathologique et inflammation.

### Exemple 34

### Interleukin 15 receptor Q13261

La séquence 54 :
F RGRHYKREFR 70 (SEQ ID NO : 54)

Anticorps contre la séquence 54 pIDSeqC, ont été préparé comme décrit dans le Exemple 1. Anticorps ont prévenu la modification de cette protéine.

### Exemple 35.

### Diacylglycerol kinase delta (QI6760) cytoplasm :

La séquence 55 et 56 :
1 FKKEKNNKNK EAHSSL (SEQ ID NO : 55)
   Predicted disorder segment: [1]-[16] length: 16 score: -0.84 ± 0.27
1 FKKEKN (SEQ ID NO: 56)
   Predicted disorder segment: [1]-[6] length: 6 score: -0.91 ± 0.00

Anticorps contre les séquences 55 et 56 pIDSeqC, ont été préparé comme décrit dans le Exemple 1. Anticorps ont prévenu la anormalité de la kinase.

### Exemple 36.

### WD repeat domain phosphoinositide-interacting protein 4 (Q9Y484)

La séquence 57
85 AREGKDSKEK L 94 (SEQ ID NO : 57)
   Predicted disorder segment: [1]-[11] length: 11 score: -0.48 ± 0.00

Anticorps contre la séquence 57 pIDSeqC, ont été préparé comme décrit dans le Exemple 1. Anticorps ont prévenu l'apoptose pathologique.

### Exemple 37.

### Retinal cone rhodopsin-sensitive cGMP 3',5'-cyclic phosphodiesterase subunit gamma Q13956

La séquence 58.
19 PRKGPPKFKQ RQTRQFKSKP PKKGVKGF 46 (SEQ ID NO : 58) Predicted disorder segment: [1]-[28] length:: 28 score: -0.84 ± 0.14

Anticorps contre la séquence 58 pIDSeqC, ont été préparé comme décrit dans le Exemple 1. Anticorps ont prévenu une dégénérescence de cellules de la rétine.

### Exemple 38.

### La SEQ ID : 11 et la SEQ : ID 18.

Anticorps contre SEQ ID : 11 et SEQ : ID 18.
(peptides mélangés ensemble 1mole : 1mole) pIDSeqC, ont été préparé comme décrit dans le Exemple 1. Anticorps ont prévenu une dégénérescence de cellules, inflammation, beta-amyloide agrégation.

### En conclusion l'invention décrite,

Un procédé destiné à provoquer des réactions immunitaires par introduction dans un organisme vivant, consistant en ce qu'il est le produit d'un ou plusieurs peptides correspondant à des séquences intrinsèquement désordonnées, déterminés par le logiciel FoldIndex©, modifiées dans une façon covalentes. Dans le composé les peptides sont polymérisés. Dans le composé les peptides appartiennent à la même protéine ou les peptides appartiennent aux protéines différentes. Composé contient une ou plusieurs séquences intrinsèquement désordonnées des protéines présentées dans exemples 2 au 37. Composé contient une ou une partie des séquences de protéines présentées dans exemples 2 au 37. Composé contient une mélange des séquences présentées dans exemples 2 au 37. Un composé selon cette invention est utilisée pour la fabrication d'un produit pour le diagnostique, un produit cosmétique ou d'un médicament pour la prévention ou thérapie des maladies dégénératives, pour l'activation du système immunitaire, la thérapie des cancers, la cardiomyopathie, l'hypertension, la congestion cérébrale, circulation de sang, régulation des lipides de sang, l'athérosclérose, dégénérescent de valve cardiaque, les maladies ophtalmiques telles que dégénérescence de la rétine, glaucome, cataracte; l'ostéoporose, et les dysfonctionnements des canaux ioniques les maladies à prions, le Parkinson, l'Alzheimer, et toute autre maladie associée à l'accumulation du beta-amyloïde telles que la dégénérescence du rein.

## Revendications

1. Procédé de fabrication d'anticorps par introduction dans un organisme vivant non-humain, d'un composé contenant un ou plusieurs peptides présentant une ou des réticulations covalentes et irréversibles entre deux mêmes peptides ou avec un peptide différent, les réticulations étant présentes sur les fonctions amines secondaires de un ou plusieurs acides aminés, modifiés de façons covalente par l'acide xanthurénique, lesdits peptides consistant en une séquence intrinsèquement désordonnée, et qui a été modifié de façon covalente irréversible sur les fonctions amines secondaires des acides aminés.

2. Le procédé selon la revendication 1 dans lequel le composé pour la fabrication d'anticorps contient des peptides qui sont polymérisés

3. Le procédé selon la revendication 1 dans lequel le composé pour la fabrication d'anticorps contient des peptides qui appartiennent à la même protéine.

4. Le procédé selon la revendication 1 dans lequel le composé pour la fabrication d'anticorps contient des peptides qui appartiennent à diverses protéines.

5. Le procédé selon la revendication 1 suivant lequel chacun des composés pour la fabrication d'anticorps contient un ou plusieurs peptides consistant en une séquence sélectionnée parmi le groupe constitué des séquences intrinsèquement désordonnées SEQ ID NO: 1 au 58.

6. Les anticorps obtenus par le procédé selon l'une des revendications 1-5, dans lequel le composé utilisé contient une ou plusieurs peptides, consistant en une séquence sélectionnée parmi le groupe constitué des séquences intrinsèquement désordonnées, SEQ ID NO: 1 au 58, **caractérisés en ce que** lesdits anticorps sont capables d'éliminer des protéines mal pliées des membranes des cellules.

7. Utilisation des anticorps selon la revendication 6 pour la fabrication d'un produit à usage diagnostique in vitro ou d'un produit cosmétique.

8. Anticorps selon la revendication 6 pour leur utilisation comme médicament.

9. Vaccin contenant le composé selon l'une des revendications 1 à 5, ledit composé contenant un ou plusieurs peptides consistant en une séquence sélectionnée parmi le groupe constitué des séquences intrinsèquement désordonnées SEQ ID NO: 1 au 58.

## Patentansprüche

1. Herstellungsverfahren von Antikörpern durch Einführung in einen lebendigen nichtmenschlichen Organismus, einer Zusammensetzung von einem oder mehreren Peptiden, die eine oder mehrere kovalente und unumkehrbare Bindungen unter denselben Peptiden oder unterschiedlichen Peptiden enthalten, diese Bindungen sind in den sekundären Aminogruppen von einer oder mehrerer Aminosäuren anwesend, die auf eine kovalente Weise durch die Xanthurensäure modifiziert wurden, die obenerwähnten Peptiden bestehen aus einer intrinsisch ungeordneten Sequenz, die auf eine kovalente und unumkehrbare Weise in Aminogruppen der sekundären Aminosäuren modifiziert wurde.

2. Das Verfahren nach dem Anspruch 1, in dem die Zusammensetzung für die Herstellung von Antikörpern, die polymerisierten Peptiden enthält.

3. Das Verfahren nach dem Anspruch 1, in dem die Zusammensetzung für die Herstellung von Antikörpern, die den Peptiden enthält die zu demselben Protein gehören.

4. Das Verfahren nach dem Anspruch 1, in dem die Zusammensetzung für die Herstellung von Antikörpern, die den Peptiden enthält die zu unterschiedlichen Proteinen gehören.

5. Das Verfahren nach dem Anspruch 1, entsprechend jede Zusammensetzung für die Herstellung von Antikörpern ein oder mehrere Peptide enthält, die aus einer der Sequenzen bestehen, die von einer der Gruppen ausgewählt ist, die intrinsisch unordentlichen Sequenzen SEQ ID NO: 1 bis 58 enthalten.

6. Die Antikörper, die durch das Verfahren nach einem der Ansprüche 1-5 erhalten sind, in denen die benutzte Zusammensetzung ein oder mehrere Peptide enthält, besteht aus einer Sequenz ausgewählt unter Gruppen, die intrinsisch unordentlichen Sequenzen SEQ ID NO: 1 bis 58 enthalten, und diese Antikörper charakterisieren sich dadurch dass sie fähig sind, die die falschgefaltete Proteine aus den Zellmembranen zu beseitigen.

7. Benutzung der Antikörper nach dem Anspruch 6 für die Herstellung eines Produktes für den diagnostischen Gebrauch in vitro oder im kosmetischen Produkt.

8. Antikörper nach dem Anspruch 6 für ihre Benutzung als Medikament.

9. Der Impfstoff, der die Zusammensetzung nach einem der Ansprüche von 1 bis 5 enthält, diese Zusammensetzung enthält ein oder mehrere Peptide, die aus einer Sequenz bestehen ausgewählt unter Gruppen, die intrinsisch unordentlichen Sequenzen SEQ ID NO: 1 bis 58 enthalten.

## Claims

1. Process of antibody preparation by introduction into a non-human living organism a composition containing one or more peptide which is covalently and irreversibly cross-linked to itself or to a different peptide, wherein said crosslinking occurs at the secondary amines of one or more amino acids, covalently modified by xanthurenic acid and wherein said peptides correspond to a intrinsically disordered sequence, which is modified in a covalent and irreversible way on the secondary amines of the amino acids.

2. Process according to claim 1 in which the composition for the preparation of antibody contains the peptides which are polymerized.

3. Process according to claim 1 in which the composition for the production of antibody contains the peptides, which belong to the same protein.

4. Process according to claim 1 in which the composition for the production of antibody contains the peptides, which belong to various proteins.

5. Process according to the claim 1 in which each of the composition for the manufacturing of antibody contains one or more peptides having a sequence selected from the group constituted of intrinsically disordered sequences SEQ ID NO: 1 to 58.

6. Antibodies obtained by the process according to any one of claims 1-5 in which the composition used contains one or more peptides having a sequence selected from the group constituted of intrinsically disordered sequences SEQ ID NO: 1 to 58, **characterized in that** the said antibodies are capable of eliminating the misfolded proteins from the cell membranes.

7. Use of antibodies according to claim 6 for manufacture of a product for diagnostic in vitro or a cosmetic product.

8. Antibodies according to claim 6 for their use as a medicine.

9. Vaccine containing the composition according to one of the claims 1 to 5, the said composition containing one or more peptides having a sequence selected from the group of intrinsically disordered sequences SEQ ID NO: 1 to 58.


### SEQUENCE LISTING

<110> Malina, Halina z Ph.D.
<120> Modification des séquences intrinsèquement desordonnées pour la préparation de Vaccins
<130> FR 0707646
<140> FR 0707646
   <141> 2007-10-31
<160> 58
<170> PatentIn version 3.5
<210> 1
   <211> 32
   <212> PRT
   <213> Homo sapiens
<400> 1
   Asn Lys Glu Tyr Gly Ser Glu Lys Lys Gly Tyr Leu Leu Lys Lys Ser
   1               5                   10                  15
   Asp Gly Ile Arg Lys Val Trp Gln Arg Arg Lys Cys Ser Val Lys Asn
               20                  25                  30
<210> 2
   <211> 20
   <212> PRT
   <213> Homo sapiens
<400> 2
   Leu Val Leu Arg Arg Lys Arg Lys Arg Met Thr Asp Pro Thr Arg Arg
   1               5                   10                  15
   Phe Phe Lys Val
               20
<210> 3
   <211> 35
   <212> PRT
   <213> Homo sapiens
<400> 3
   Asn Lys Glu Tyr Gly Ser Glu Lys Lys Gly Tyr Leu Leu Lys Lys Ser
   1               5                   10                  15
   Asp Gly Ile Arg Lys Val Trp Gln Arg Arg Lys Cys Ser Val Lys Asn
   20 25 30
   Gly Ile Leu
   35
<210> 4
   <211> 11
   <212> PRT
   <213> Homo sapiens
<400> 4
   Thr Lys Lys Phe Gly Trp Val Lys Lys Tyr Val
   1 5 10
<210> 5
   <211> 8
   <212> PRT
   <213> Homo sapiens
<400> 5
   Val Lys Lys Ile Pro Lys Lys Pro
   1 5
<210> 6
   <211> 16
   <212> PRT
   <213> Homo sapiens
<400> 6
   Phe His Lys Gln Asn Lys Met Leu Lys Lys Asp Lys Met Phe His Phe
   1 5 10 15
<210> 7
   <211> 14
   <212> PRT
   <213> Homo sapiens
<400> 7
   Arg Asp Ile Tyr Glu Thr Asp Tyr Tyr Arg Lys Gly Gly Lys
   1 5 10
<210> 8
   <211> 93
   <212> PRT
   <213> Homo sapiens
<400> 8
   Asn Cys Gln Lys Leu Thr Lys Ile Ile Cys Ala Gln Gln Cys Ser Gly
   1 5 10 15
   Arg Cys Arg Gly Lys Ser Pro Ser Asp Cys Cys His Asn Gln Cys Ala
   20 25 30
   Ala Gly Cys Thr Gly Pro Arg Glu Ser Asp Cys Leu Val Cys Arg Lys
   35 40 45
   Phe Arg Asp Glu Ala Thr Cys Lys Asp Thr Cys Pro Pro Leu Met Leu
   50 55 60
   Tyr Asn Pro Thr Thr Tyr Gln Met Asp Val Asn Pro Glu Gly Lys Tyr
   65 70 75 80
   Ser Phe Gly Ala Thr Cys Val Lys Lys Cys Pro Arg Asn
   85 90
<210> 9
   <211> 27
   <212> PRT
   <213> Homo sapiens
<400> 9
   Met Ser Glu Arg Lys Glu Gly Arg Gly Lys Gly Lys Gly Lys Lys Lys
   1 5 10 15
   Glu Arg Gly Ser Gly Lys Lys Pro Glu Ser Ala
   20 25
<210> 10
   <211> 21
   <212> PRT
   <213> Homo sapiens
<400> 10
   Glu Lys Lys Ile Glu Met Val Arg Ala Tyr Arg Glu Lys Ile Glu Lys
   1 5 10 15
   Glu Leu Glu Ala Val
   20
<210> 11
   <211> 10
   <212> PRT
   <213> Homo sapiens
<400> 11
   Phe Lys Ser Gly Leu Lys Tyr Lys Lys Gly
   1 5 10
<210> 12
   <211> 22
   <212> PRT
   <213> Homo sapiens
<400> 12
   Val Arg Phe Leu Asp Lys Lys His Arg Asn His Tyr Arg Val Tyr Asn
   1 5 10 15
   Leu Cys Ser Glu Arg Ala
   20
<210> 13
   <211> 15
   <212> PRT
   <213> Homo sapiens
<400> 13
   Trp Cys Lys Arg Gly Arg Lys Gln Cys Lys Thr His Pro His Phe
   1 5 10 15
<210> 14
   <211> 23
   <212> PRT
   <213> Homo sapiens
<400> 14
   Phe Gln Lys Ala Lys Glu Arg Leu Glu Ala Lys His Arg Glu Arg Met
   1 5 10 15
   Ser Gln Val Met Arg Glu Trp
   20
<210> 15
   <211> 22
   <212> PRT
   <213> Homo sapiens
<400> 15
   Ala Lys Ala Asp Lys Ile Lys Leu Ala Leu Glu Lys Leu Lys Glu Ala
   1 5 10 15
   Lys Val Lys Lys Leu Val
   20
<210> 16
   <211> 43
   <212> PRT
   <213> Homo sapiens
<400> 16
   Ala Leu Lys Lys Tyr Gln Thr Glu Gln Arg Ser Lys Gly Asp Ala Leu
   1 5 10 15
   Asp Lys Cys Gln Ala Glu Leu Lys Lys Leu Arg Lys Lys Ser Gln Gly
   20 25 30
   Ser Lys Asn Pro Gln Lys Tyr Ser Asp Lys Glu
   35 40
<210> 17
   <211> 12
   <212> PRT
   <213> Homo sapiens
<400> 17
   Asn Arg Lys Asp Phe Lys Ile Asp Arg Lys Lys Ala
   1 5 10
<210> 18
   <211> 10
   <212> PRT
   <213> Homo sapiens
<400> 18
   Pro Val Lys Lys Arg Lys Arg Lys Cys Leu
   1 5 10
<210> 19
   <211> 31
   <212> PRT
   <213> Homo sapiens
<400> 19
   Thr Arg Leu Phe Gly Leu Thr Lys Pro Lys Glu Lys Lys Glu Lys Lys
   1 5 10 15
   Lys Lys Asn Lys Thr Ser Arg Ser Gln Pro Gly Asp Gly Pro Ala
   20 25 30
<210> 20
   <211> 46
   <212> PRT
   <213> Homo sapiens
<400> 20
   Phe Ser Tyr Ile Lys Asn Lys Met Ser Ser Ser Lys Lys Ser Lys Glu
   1 5 10 15
   Lys Glu Lys Glu Lys Asp Lys Ile Lys Glu Lys Glu Lys Asp Ser Lys
   20 25 30
   Asp Lys Glu Lys Asp Lys Lys Thr Val Asn Gly His Thr Phe
   35 40 45
<210> 21
   <211> 18
   <212> PRT
   <213> Homo sapiens
<400> 21
   Asn Thr Asp Arg Ser Cys Arg Lys Lys Asn Lys Gly Val Glu Arg Lys
   1 5 10 15
   Gly Glu
<210> 22
   <211> 38
   <212> PRT
   <213> Homo sapiens
<400> 22
   Ala Ser Val Ser Ser Gly Arg Lys Ser Pro His Ser Lys Ser Pro Ala
   1 5 10 15
   Glu Gln Arg Glu Arg Lys Ser Leu Ala Asp Asp Lys Lys Lys Val Lys
   20 25 30
   Asn Leu Gly Tyr Arg Asp
   35
<210> 23
   <211> 20
   <212> PRT
   <213> Homo sapiens
<400> 23
   Glu Glu Ser Arg Val Arg Arg His Lys His Ser Ser Glu Ser Pro Gly
   1 5 10 15
   Arg Asp Lys Gly
   20
<210> 24
   <211> 7
   <212> PRT
   <213> Homo sapiens
<400> 24
   Phe Leu Arg Arg Lys Arg Asp
   1               5
<210> 25
   <211> 11
   <212> PRT
   <213> Homo sapiens
<400> 25
   Ala Arg Lys Glu Lys Lys Gly Lys His Lys Gly
   1               5                   10
<210> 26
   <211> 11
   <212> PRT
   <213> Homo sapiens
<400> 26
   Asn Lys Gln Asn Gln Lys Lys Lys Arg Lys Val
   1               5                   10
<210> 27
   <211> 15
   <212> PRT
   <213> Homo sapiens
<400> 27
   Glu Arg Glu Arg Ala Arg Arg Arg Glu Glu Arg Arg Lys Arg Phe
   1               5                   10                  15
<210> 28
   <211> 19
   <212> PRT
   <213> Homo sapiens
<400> 28
   Ala Ser Lys Thr Lys Thr Lys Lys Lys His Phe Val Ala Gln Lys Val
   1               5                   10                  15
   Lys Leu Phe
<210> 29
   <211> 19
   <212> PRT
   <213> Homo sapiens
<400> 29
   Glu Met His Asn Ile Leu Lys Lys Tyr His Gln Tyr Ile Val Glu Cys
   1 5 10 15
   His Gly Ile
<210> 30
   <211> 16
   <212> PRT
   <213> Homo sapiens
<400> 30
   Ala Lys Lys Lys Ala Ala His Ala Ala Lys Thr Val Lys His Gly Ala
   1 5 10 15
<210> 31
   <211> 25
   <212> PRT
   <213> Homo sapiens
<400> 31
   Val Lys His Arg Lys Gln Gln Val Leu Glu Thr Val Ala Gly Lys Arg
   1 5 10 15
   Ser Tyr Arg Leu Ser Met Lys Val Lys
   20 25
<210> 32
   <211> 14
   <212> PRT
   <213> Homo sapiens
<400> 32
   Glu Lys Tyr Lys Ala Lys Ser Arg Ser Pro Gly Ser Pro Val
   1 5 10
<210> 33
   <211> 9
   <212> PRT
   <213> Homo sapiens
<400> 33
   Leu Arg Glu Lys Ser Lys Lys Tyr Ser
   1 5
<210> 34
   <211> 11
   <212> PRT
   <213> Homo sapiens
<400> 34
   Leu Arg Glu Lys Ser Lys Lys Tyr Ser Asp Val
   1               5                   10
<210> 35
   <211> 11
   <212> PRT
   <213> Homo sapiens
<400> 35
   Glu Arg Lys Gln Lys Gly Ser Glu Glu Asn Leu
   1               5                   10
<210> 36
   <211> 6
   <212> PRT
   <213> Homo sapiens
<400> 36
   Glu Arg Lys Gln Lys Gly
   1               5
<210> 37
   <211> 27
   <212> PRT
   <213> Homo sapiens
<400> 37
   Pro Gly Lys Ser Pro Ser Lys Lys Lys Lys Lys Phe Arg Thr Pro Ser
   1               5                   10                  15
   Phe Leu Lys Lys Ser Lys Lys Lys Ser Asp Ser
               20                  25
<210> 38
   <211> 23
   <212> PRT
   <213> Homo sapiens
<400> 38
   Gly Lys Ser Pro Ser Lys Lys Lys Lys Lys Phe Arg Thr Pro Ser Phe
   1               5                   10                  15
   Leu Lys Lys Ser Lys Lys Lys
               20
<210> 39
   <211> 19
   <212> PRT
   <213> Homo sapiens
<400> 39
   Val Ser Lys Lys Glu Tyr Glu Glu Asp Gly Ala Arg Ser Ile His Arg
   1               5                   10                  15
   Lys Thr Phe
<210> 40
   <211> 22
   <212> PRT
   <213> Homo sapiens
<400> 40
   Met Tyr Arg Arg Lys Ser Lys Gln Ala Leu Arg Asp Tyr Lys Lys Val
   1               5                   10                  15
   Gln Ile Gln Leu Glu Asn
               20
<210> 41
   <211> 52
   <212> PRT
   <213> Homo sapiens
<400> 41
   Pro Lys Lys Leu Ser Gly Lys Ser Lys Ser Gly Arg Ser Leu Asn Glu
   1               5                   10                  15
   Glu Leu Gly Asp Glu Asp Ser Glu Lys Lys Arg Lys Gly Ala Phe Phe
               20                  25                  30
   Ser Trp Ser Arg Thr Arg Ser Thr Gly Arg Ser Gln Lys Lys Arg Glu
           35                  40                  45
   His Gly Asp His
       50
<210> 42
   <211> 37
   <212> PRT
   <213> Homo sapiens
<400> 42
   Asn Ser Ile Lys Ile Lys Gly Glu Asn Gly Lys Asn Ala Arg Asp Pro
   1               5                   10                  15
   Arg Leu Ser Lys Arg Glu Glu Ser Ile Ala Lys Ile Gly Lys Lys Lys
   20 25 30
   Tyr Gln Lys Ile Asn
   35
<210> 43
   <211> 23
   <212> PRT
   <213> Homo sapiens
<400> 43
   Glu Lys Lys Asp Gly Asp Lys Lys Lys Lys Lys Lys Ile Lys Glu Lys
   1 5 10 15
   Tyr Ile Asp Gln Glu Glu Leu
   20
<210> 44
   <211> 16
   <212> PRT
   <213> Homo sapiens
<400> 44
   Val Gln Gly Lys Thr Lys Arg Pro Val Asp Leu Lys Ile Lys Asn Leu
   1 5 10 15
<210> 45
   <211> 14
   <212> PRT
   <213> Homo sapiens
<400> 45
   Val Ser Arg Leu Val Lys Lys Ile Pro Lys Lys Pro Pro Ala
   1 5 10
<210> 46
   <211> 15
   <212> PRT
   <213> Homo sapiens
<400> 46
   Trp Cys Lys Arg Gly Arg Lys Gln Cys Lys Thr His Pro His Phe
   1 5 10 15
<210> 47
   <211> 16
   <212> PRT
   <213> Homo sapiens
<400> 47
   Phe Gln Lys Ala Lys Glu Arg Leu Glu Ala Lys His Arg Glu Arg Met
   1 5 10 15
<210> 48
   <211> 23
   <212> PRT
   <213> Homo sapiens
<400> 48
   Leu Arg Lys Asn Lys Asn Ile Asp Asn Phe Leu Asn Arg Tyr Glu Lys
   1 5 10 15
   Ile Val Lys Lys Ile Arg Gly
   20
<210> 49
   <211> 8
   <212> PRT
   <213> Homo sapiens
<400> 49
   Glu Lys Lys Lys Lys Ile Thr Val
   1 5
<210> 50
   <211> 16
   <212> PRT
   <213> Homo sapiens
<400> 50
   Asn Arg Lys Lys His Arg Arg Lys Lys Ser Thr Ser Asn Phe Lys Ala
   1 5 10 15
<210> 51
   <211> 11
   <212> PRT
   <213> Homo sapiens
<400> 51
   Phe Arg Gly Arg His Tyr Lys Arg Glu Phe Arg
   1 5 10
<210> 52
   <211> 8
   <212> PRT
   <213> Homo sapiens
<400> 52
   Leu Arg Ile Lys Lys Lys Lys Glu
   1 5
<210> 53
   <211> 11
   <212> PRT
   <213> Homo sapiens
<400> 53
   His Arg Arg Cys Lys His Arg Thr Gly Lys Ala
   1 5 10
<210> 54
   <211> 11
   <212> PRT
   <213> Homo sapiens
<400> 54
   Phe Arg Gly Arg His Tyr Lys Arg Glu Phe Arg
   1 5 10
<210> 55
   <211> 16
   <212> PRT
   <213> Homo sapiens
<400> 55
   Phe Lys Lys Glu Lys Asn Asn Lys Asn Lys Glu Ala His Ser Ser Leu
   1 5 10 15
<210> 56
   <211> 6
   <212> PRT
   <213> Homo sapiens
<400> 56
   Phe Lys Lys Glu Lys Asn
   1 5
<210> 57
   <211> 11
   <212> PRT
   <213> Homo sapiens
<400> 57
   Ala Arg Glu Gly Lys Asp Ser Lys Glu Lys Leu
   1               5                   10
<210> 58
   <211> 28
   <212> PRT
   <213> Homo sapiens
<400> 58
   Pro Arg Lys Gly Pro Pro Lys Phe Lys Gln Arg Gln Thr Arg Gln Phe
   1               5                   10                  15
   Lys Ser Lys Pro Pro Lys Lys Gly Val Lys Gly Phe
               20                  25